(19) Europäisches Patentamt
European Patent Office
Office européen des brevets

(11) **EP 4 361 572 B1**

(12) **EUROPÄISCHE PATENTSCHRIFT**

(45) Veröffentlichungstag und Bekanntmachung des Hinweises auf die Patenterteilung:
**25.02.2026 Patentblatt 2026/09**

(21) Anmeldenummer: **22204507.2**

(22) Anmeldetag: **28.10.2022**

(51) Internationale Patentklassifikation (IPC):
**G01D 11/30** (2006.01)   **G01N 33/00** (2006.01)

(52) Gemeinsame Patentklassifikation (CPC):
**G01D 11/30; G01N 33/0009**

(54) **ANORDNUNG UND/ODER ANALYSEGERÄT MIT MITTELWERTBILDUNG**

AVERAGING ARRANGEMENT AND/OR ANALYZER

DISPOSITIF ET/OU ANALYSEUR AVEC MOYENNAGE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(43) Veröffentlichungstag der Anmeldung:
**01.05.2024 Patentblatt 2024/18**

(73) Patentinhaber: **Siemens Aktiengesellschaft 80333 München (DE)**

(72) Erfinder:
• **Baader, Mathias**
  **76437 Rastatt (DE)**
• **Sperrfechter, Frank**
  **76470 Ötigheim (DE)**
• **Weida, Simon**
  **76131 Karlsruhe (DE)**

(74) Vertreter: **Siemens Patent Attorneys Postfach 22 16 34 80506 München (DE)**

(56) Entgegenhaltungen:
**DE-A1- 102012 211 039    US-A- 6 015 533 US-A1- 2010 050 738**

EP 4 361 572 B1

**Beschreibung**

Hintergrund

Die vorliegende Offenbarung bezieht sich auf eine Anordnung und/oder auf ein Analysegerät zur Bestimmung von Gaskonzentrationen. Insbesondere bezieht sich die vorliegende Offenbarung auf eine solche Anordnung und/oder ein solches Analysegerät zum Einsatz in einer Verbrennungsvorrichtung. Eine zu bestimmende Gaskonzentration kann beispielsweise eine Konzentration an gasförmigem Sauerstoff sein.

Industrielle Prozesse verwenden Energiewandlung durch Verbrennung, um Dampf und/oder Wärme für einen industriellen Prozess zu erzeugen. Dazu brennt im Feuerraum einer Verbrennungsvorrichtung im Betrieb eine Flamme eines Wärmeerzeugers. Der Wärmeerzeuger tauscht die Wärmeenergie der heissen Brenngase in ein anderes Fluid wie beispielsweise Wasser. Mit dem warmen Wasser wird beispielsweise eine Warmwasserheizungsanlage betrieben und/oder Trinkwasser erwärmt. Gemäss einer anderen Ausführungsform kann mit der Wärmeenergie der heissen Brennstoffe und/oder Brenngase ein Gut beispielsweise in einem industriellen Prozess erhitzt werden. Gemäss einer weiteren Ausführungsform ist der Wärmeerzeuger Teil einer Anlage mit Kraft-Wärme-Kopplung, beispielsweise ein Motor einer solchen Anlage. Gemäss einer anderen Ausführungsform ist der Wärmeerzeuger eine Gasturbine. Ferner kann der Wärmeerzeuger der Erhitzung von Wasser in einer Anlage zur Gewinnung von Lithium und/oder Lithiumkarbonat dienen. Die Abgase werden aus dem Feuerraum beispielsweise über einen Abgaskamin und/oder einen Rauchgaskamin und/oder einen Schornstein abgeführt.

Einige solche Prozesse beinhalten den Betrieb eines Ofens oder Kessels. Während die Verbrennung eine kostengünstige Energiewandlung darstellt, wird häufig versucht, die Verbrennungseffizienz innerhalb eines Prozesses zu maximieren. Die Maximierung der Verbrennungseffizienz ist unter anderem eine Folge der resultierenden Abgase und/oder Rauchgase, die das System verlassen. Jene Abgase und/oder Rauchgase unterliegen bisweilen Vorschriften bezüglich der Emissionen schädlicher Gase. Mithin besteht ein Ziel der Optimierung darin, die Verbrennungseffizienz bestehender Öfen und/oder Kessel zu maximieren. Damit einher geht eine Reduktion der Produktion von Treibhausgasen und anderen schädlichen Nebenprodukten.

Ein weiteres Ziel ist der Optimierung für verschiedene Brennstoffe und/oder Brenngase. Insbesondere geht es dabei um Brennstoffe und/oder um Brenngase, welche Wasserstoffgase umfassen. Vorteilhaft geht es um Brennstoffe und/oder um Brenngase, welche bei 293 Kelvin mehr als zwanzig Volumenprozent Wasserstoff umfassen. In einigen Fällen liegt bei 293 Kelvin der Anteil an Wasserstoffgas bei fünfzig Volumenprozent oder gar bei siebzig Volumenprozent.

Die Verbrennungseffizienz kann optimiert werden, indem auf den Sauerstoffgehalt in den aus einem Verbrennungsprozess stammenden Abgasen und/oder Rauchgasen geregelt wird. Damit wird die Oxidation der Verbrennungsnebenprodukte weitgehend sicherstellt.

In-situ- oder In-Prozess-Analysegeräte werden können beim Überwachen und/oder Optimieren und/oder Steuern eines laufenden Verbrennungsprozesses eingesetzt werden. Gängige Analysegeräte umfassen eine Sensoreinheit. Die Sensoreinheit wird auf hohe Temperaturen erhitzt. Sie arbeitet direkt in der Verbrennungszone oder nahe der Verbrennungszone des Ofens oder Kessels.

Bekannte Analysegeräte verwenden typischerweise einen auf Zirconiumdioxid basierenden Sauerstoffsensor. Der Sauerstoffsensor ist an einem Ende einer Sonde angeordnet, die in einen Rauchgasstrom eingeführt wird. Wenn das Abgas und/oder Rauchgas in das Analysegerät strömt, diffundiert es durch einen Filter oder Diffusor in die Nähe des auf Zirconiumdioxid basierenden Sauerstoffsensors. Es gibt keine Pumpen und/oder andere strömungsinduzierende Vorrichtungen, die verwendet werden, um den Probenfluss in das Analysegerät zu leiten. Stattdessen dringt das Gas passiv durch den Diffusor. Der Sensor liefert ein elektrisches Signal, das eine im Abgas und/oder Rauchgas vorhandene Sauerstoffmenge angibt.

Der auf Zirconiumdioxid basierende Sauerstoffsensor bietet eine potentiometrische Indikation. Die potentiometrische Indikation gilt als zuverlässige Sauerstoffmessung in Verbrennungsumgebungen. Sie ermöglicht eine effiziente und/oder sichere Prozesssteuerung. Typischerweise wird eine einzelne Sonde in den Prozess beispielsweise in den Abgaskamin und/oder in den Rauchgaskamin und/oder in den Schornstein eingeführt. Eine prozentuale Sauerstoffmessung wird verwendet, um die Verbrennungseffizienz in kleinen Kesseln und/oder Öfen zu optimieren. Bei grossen Anlagen trifft der Betreiber vielfach auf eine Abgasschichtung und/oder Rauchgasschichtung. Die Abgasschichtung und/oder Rauchgasschichtung umfasst eine Vielzahl an Schichten mit jeweils unterschiedlicher Sauerstoffkonzentration.

Um Schichtungsinformationen zu erhalten, können Betreiber für einen effizienten und sicheren Betrieb mehrere Sonden in den Abgaskamin und/oder in den Rauchgaskamin und/oder in den Schornstein einbauen. Es können in einzelnen Fällen bis zu sechzehn solche Sonden eingebaut werden.

Ein typisches In-situ- oder In-Prozess-Analysegerät mit einem potentiometrischen Sensor auf Basis von Zirconiumdioxid bietet ein Einzelpunktsauerstoffmessung. Solche Analysegeräte dienen zur Optimierung der Verbrennungseffizienz in Kraftwerken und/oder Verbrennungsanlagen und/oder Raffinerien und/oder Chemieanlagen und/oder kleinen Verbrennungsanlagen. Wie oben beschrieben, weisen grosse Abgaskamine eine erhebliche Abgasschichtung mit einer Vielzahl unterschiedlicher Konzentrationsschichten im Abgas auf.

Ferner weisen grosse Rauchgaskamine eine erhebliche Rauchgasschichtung mit einer Vielzahl unterschiedlicher Konzentrationsschichten im Rauchgas auf. Darüber hinaus weisen grosse Schornsteine eine erhebliche Abgasschichtung und/oder Rauchgasschichtung mit einer Vielzahl unterschiedlicher Konzentrationsschichten im Abgas und/oder Rauchgas auf.

In solchen Fällen ist es bei solch grossen Verbrennungsanwendungen üblich, mehrere Sauerstofferfassungssonden zu verwenden. Die Verwendung solcher Sonden erhöht jedoch die Komplexität und die Fehleranfälligkeit des gesamten Verbrennungsautomatisierungssystems. Beispielsweise erfordert jedes Analysegerät Strom- und/oder Signalkabel, Kalibriergasleitungen und eine Halterung.

Eine Alternative für einige grosse Verbrennungsanwendungen zur Bereitstellung von Schichtungsinformationen ist die Verwendung eines Sauerstoffsensors basierend auf einem abstimmbaren Diodenlaser. Solche Sensoren werden in Anwendungen verwendet, um durchschnittliche Sauerstoffkonzentrationen bereitzustellen. Solche Systeme weisen nicht den Vorteil einer regelmässigen In-situ-Kalibrierung auf. Ferner beruhen solche abstimmbaren Diodenlaser auf optischer Strahlung, die durch das Abgas und/oder Rauchgas hindurchtritt. Abstimmbare Diodenlaser unterliegen dann Einschränkungen, wenn das Abgas und/oder Rauchgas teilweise oder vollständig undurchsichtig ist.

Ein publiziertes Gebrauchsmuster CN2685875Y aus China wurde am 17. Juli 2003 eingereicht. Das Gebrauchsmuster CN2685875Y wurde am 16. März 2005 publiziert. Die Schrift CN2685875Y befasst sich mit einem integralen Rauchanalysegerät auf Basis von Zirconiumdioxid. Die Schrift CN2685875Y offenbart ein Analysegerät mit einer Messspitze.

An einem ersten Ende der Messspitze sind ein Sensor auf Basis von Zirconiumdioxid, eine Heizvorrichtung und ein Temperatursensor angebracht. An einem zweiten Ende der Messspitze befinden sich zwei Gasanschlüsse sowie ein Gehäuse. Aus dem Gehäuse ragt ein Auslass.

Eine internationale Patentanmeldung WO2022/064271A1 wurde eingereicht am 11. Dezember 2020 durch ROSEMOUNT INC. Die Anmeldung wurde veröffentlicht am 31. März 2022. WO2022/064271A1 nimmt eine Priorität vom 24. September 2020 in Anspruch. Die Anmeldung WO2022/064271A1 befasst sich mit einem In-situ-Analysegerät mit Mittelwertbildung. WO2022/064271A1 offenbart ein Analysegerät mit einer Messspitze. Die Messspitze weist ein erstes und ein zweites Ende auf. Zwischen dem ersten und dem zweiten Ende der Messspitze befinden sich mehrere Öffnungen. Nahe dem zweiten Ende der Messspitze befinden sich eine Sensoreinheit und ein Flansch zur Befestigung des Analysegerätes. Mithin strömt Gas durch jede Öffnung der Vielzahl an Öffnungen in Richtung der Sensoreinheit. Durch die Vielzahl an Öffnungen erfolgt eine Mittelung von Gaskonzentrationen.

Eine Patentanmeldung DE102012211039A1 wurde eingereicht am 27. Juni 2012 durch die Robert Bosch GmbH, 70469, Stuttgart. Die Anmeldung wurde veröffentlicht am 2. Januar 2014. DE102012211039A1 behandelt einen Gassensor für Russ.

Das Patent US6015533A ist erteilt am 18. Januar 2000 an Motorola Inc (Schaumburg, IL). Es behandelt ein Sensorgehäuse für einen kalorimetrischen Sensor. Anmeldetag von US6015533A ist der 14. November 1997.

Eine Patentanmeldung US2010/050738A1 wurde eingereicht am 26. August 2008 Die Anmeldung wurde veröffentlicht am 4. März 2010. US2010/050738A1 behandelt eine Sensoranordnung mit einem thermisch isolierenden Gehäuse.

Ziel der vorliegenden Offenbarung ist es, eine Anordnung bereitzustellen, welche eine Gasanalyse an einer Verbrennungsvorrichtung ermöglicht. Dabei sind verschiedene Konzentrationen solcher Gase derart zu Mitteln, dass ein aussagekräftiges Ergebnis erhalten wird. Insbesondere soll das Ergebnis eine Steuerung und/oder Regelung der Verbrennungsvorrichtung ermöglichen.

Zusammenfassung

Die obengenannte Aufgabe wird durch eine Anordnung mit In-situ und/oder In-Prozess Mittelwertbildung , wie in Anspruch 1 definiert, gelöst. Die Anordnung umfasst ein Gehäuse und eine Sensoreinheit. Die Sensoreinheit ist an dem Gehäuse befestigt.

Das Gehäuse kann insbesondere als Messspitze ausgebildet sein. Das Gehäuse umfasst ein erstes und ein zweites Ende. Das erste Ende des Gehäuses ist verschieden vom zweiten Ende des Gehäuses. Das erste Ende des Gehäuses ist dem zweiten Ende des Gehäuses gegenüberliegend angeordnet. Insbesondere kann die Messspitze ein erstes und ein zweites Ende umfassen. Das erste Ende der Messspitze ist verschieden vom zweiten Ende der Messspitze. Das erste Ende der Messspitze ist dem zweiten Ende der Messspitze gegenüberliegend angeordnet.

Das erste Ende des Gehäuses wird vorzugsweise anhand eines Flansches an einer Seitenwand eines Abgaskamins und/oder Rauchgaskamins und/oder Schornsteins angeordnet. Es ist vorgesehen, dass das zweite Ende des Gehäuses in den Abgaskamin und/oder in den Rauchgaskamin und/oder in den Schornstein hineinragt. Insbesondere kann das erste Ende der Messspitze beispielsweise anhand eines Flansches an einer Seitenwand des Abgaskamins und/oder Rauchgaskamins und/oder Schornsteins angeordnet sein. Ferner kann das zweite Ende der Messspitze in den Abgaskamin und/oder in den Rauchgaskamin und/oder in den Schornstein hineinragen.

Die Sensoreinheit ist in dem Gehäuse angeordnet. Die Sensoreinheit ist nahe dem zweiten Ende des Gehäuses angeordnet. Das heisst, dass die Sensoreinheit näher am zweiten Ende des Gehäuses angeordnet ist als am ersten Ende des Gehäuses. Mit anderen Worten, ein erster Abstand der Sensoreinheit vom ersten Ende des Gehäuses ist grösser als ein zweiter Abstand der Sensoreinheit vom zweiten Ende des Gehäuses.

Insbesondere kann die Sensoreinheit in der Messspitze angeordnet und befestigt sein. Die Sensoreinheit ist nahe dem zweiten Ende der Messspitze angeordnet. Das heisst, dass die Sensoreinheit näher am zweiten Ende der Messspitze angeordnet ist als am ersten Ende der Messspitze. Mit anderen Worten, ein erster Abstand der Sensoreinheit vom ersten Ende der Messspitze ist grösser als ein zweiter Abstand der Sensoreinheit vom zweiten Ende der Messspitze.

Zwischen dem ersten Ende des Gehäuses und dem zweiten Ende des Gehäuses ist eine Vielzahl an Einlassöffnungen angeordnet. Die Abstände zwischen den einzelnen Einlassöffnungen des Gehäuses können dabei unterschiedlich sein. Ebenso können die Einlassöffnungen des Gehäuses unterschiedliche Öffnungsquerschnitte aufweisen.

Insbesondere kann zwischen dem ersten Ende der Messspitze und dem zweiten Ende der Messspitze eine Vielzahl an Einlassöffnungen angeordnet sein. Die Abstände zwischen den einzelnen Einlassöffnungen der Messspitze können dabei unterschiedlich sein. Ebenso können die Einlassöffnungen der Messspitze unterschiedliche Öffnungsquerschnitte aufweisen.

Nahe dem zweiten Ende des Gehäuses oder am zweiten Ende des Gehäuses ist eine Auslassöffnung angeordnet. Das heisst, dass die Auslassöffnung näher am zweiten Ende des Gehäuses als am ersten Ende des Gehäuses liegt. Mit anderen Worten, ein erster Abstand der Auslassöffnung vom ersten Ende des Gehäuses ist grösser als ein zweiter Abstand der Auslassöffnung vom zweiten Ende des Gehäuses.

Die Auslassöffnung des Gehäuses ist vorzugsweise eine Auslassöffnung für Abgase und/oder Rauchgase.

Die Auslassöffnung des Gehäuses ist verschieden von jedem Einlass der Vielzahl an Einlassöffnungen des Gehäuses.

Vorteilhaft umfasst das Gehäuse genau eine Auslassöffnung für Abgase und/oder Rauchgase.

In einer ersten Ausführungsform sind die Einlassöffnungen an einer ersten Seite des Gehäuses angeordnet. Die Auslassöffnung ist an einer zweiten Seite des Gehäuses angeordnet, wobei die zweite Seite der ersten Seite gegenüberliegt. Die erste Seite des Gehäuses ist verschieden vom ersten Ende und vom zweiten Ende des Gehäuses. Die zweite Seite des Gehäuses ist verschieden vom ersten Ende und vom zweiten Ende des Gehäuses.

In einer zweiten Ausführungsform ist die Auslassöffnung des Gehäuses am zweiten Ende des Gehäuses angeordnet. Diese Auslassöffnung kann beispielsweise eine Bohrung durch das zweite Ende des Gehäuses umfassen oder sein.

In einer dritten Ausführungsform umfasst das zweite Ende des Gehäuses einen abgeschrägten Abschnitt. Der abgeschrägte Abschnitt des Gehäuses umfasst die Auslassöffnung. Die Auslassöffnung des abgeschrägten Endes des Gehäuses kann beispielsweise eine Bohrung durch den abgeschrägten Abschnitt des Gehäuses umfassen oder sein.

Nahe dem zweiten Ende der Messspitze oder am zweiten Ende der Messspitze kann eine Auslassöffnung angeordnet sein. Das heisst, dass die Auslassöffnung näher am zweiten Ende der Messspitze als am ersten Ende der Messspitze liegt. Mit anderen Worten, ein erster Abstand der Auslassöffnung vom ersten Ende der Messspitze ist grösser als ein zweiter Abstand der Auslassöffnung vom zweiten Ende der Messspitze.

Die Auslassöffnung der Messspitze ist vorzugsweise eine Auslassöffnung für Abgase und/oder Rauchgase.

Die Auslassöffnung der Messspitze ist verschieden von jedem Einlass der Vielzahl an Einlassöffnungen der Messspitze.

Vorteilhaft umfasst die Messspitze genau eine Auslassöffnung für Abgase und/oder Rauchgase.

In einer ersten Ausführungsform sind die Einlassöffnungen an einer ersten Seite der Messspitze angeordnet. Die Auslassöffnung ist an einer zweiten Seite der Messspitze angeordnet, wobei die zweite Seite der ersten Seite gegenüberliegt. Die erste Seite der Messspitze ist verschieden vom ersten Ende und vom zweiten Ende der Messspitze. Die zweite Seite der Messspitze ist verschieden vom ersten Ende und vom zweiten Ende der Messspitze.

In einer zweiten Ausführungsform ist die Auslassöffnung der Messspitze am zweiten Ende der Messspitze angeordnet. Diese Auslassöffnung kann beispielsweise eine Bohrung durch das zweite Ende der Messspitze umfassen oder sein.

In einer dritten Ausführungsform umfasst das zweite Ende der Messspitze einen abgeschrägten Abschnitt. Der abgeschrägte Abschnitt der Messspitze umfasst die Auslassöffnung. Die Auslassöffnung des abgeschrägten Endes der Messspitze kann beispielsweise eine Bohrung durch den abgeschrägten Abschnitt der Messspitze umfassen oder sein.

Kurze Beschreibung der Zeichnungen

Verschiedene Merkmale werden dem Fachmann aus der folgenden detaillierten Beschreibung der offenbarten nicht einschränkenden Ausführungsformen ersichtlich. Die Zeichnungen, die der detaillierten Beschreibung beiliegen, können kurz wie folgt beschrieben werden:

FIG 1 zeigt schematisch eine Anordnung und/oder ein Analysegerät zur mittelnden Bestimmung einer Gaskonzentration in einer Verbrennungsvorrichtung.

FIG 2 zeigt schematisch eine Anordnung und/oder ein Analysegerät zur mittelnden Bestimmung einer Gaskonzentration mit einer seitwärts gerichteten Auslassöffnung.

FIG 3 zeigt schematisch eine Anordnung und/oder ein Analysegerät zur mittelnden Bestimmung einer Gaskonzentration mit einer Auslassöffnung durch ein abgeschrägtes Ende.

Detaillierte Beschreibung

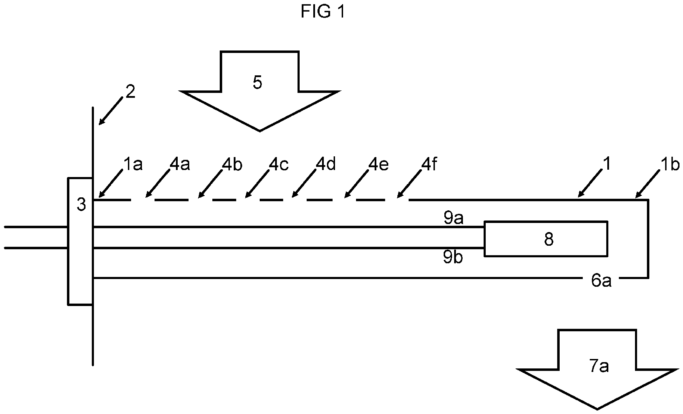

In FIG 1 ist eine Anordnung und/oder ein Analysegerät gemäss der vorliegenden Offenbarung veranschaulicht. Die Anordnung und/oder das Analysegerät umfasst ein Gehäuse 1. Das Gehäuse 1 umfasst ein erstes Ende 1a und ein zweites Ende 1b. Das erste Ende 1a des Gehäuses 1 ist verschieden vom zweiten Ende 1b des Gehäuses 1. Das erste Ende 1a liegt vorzugsweise dem zweiten Ende 1b des Gehäuses 1 gegenüber.

In einer Ausführungsform sind das erste Ende 1a des Gehäuses 1 und das zweite Ende 1b des Gehäuses 1 mindestens dreissig Millimeter voneinander beabstandet. Das erste Ende 1a und das zweite Ende 1b des Gehäuses 1 können mindestens sechzig Millimeter voneinander beabstandet sein. Das erste Ende 1a und das zweite Ende 1b des Gehäuses 1 können sogar mindestens einhundert Millimeter voneinander beabstandet sein.

Am ersten Ende 1a des Gehäuses 1 kann das Gehäuse 1 mit einer Wand 2 eines Abgaskamins und/oder Rauchgaskamins und/oder Schornsteins verbunden werden. Die Verbindung zwischen dem Gehäuse 1 und der Wand 2 kann beispielsweise anhand eines Flansches 3 erfolgen. Insbesondere kann das Gehäuse 1 anhand des Flansches 3 an der Wand 2 befestigt sein.

Insbesondere kann das Gehäuse 1 mit einer Seitenwand 2 eines Abgaskamins und/oder Rauchgaskamins und/oder Schornsteins verbunden werden. Die Verbindung zwischen dem Gehäuse 1 und der Seitenwand 2 kann beispielsweise anhand eines Flansches 3 erfolgen. Insbesondere kann das Gehäuse 1 anhand des Flansches 3 an der Seitenwand 2 befestigt sein.

Das zweite Ende 1b des Gehäuses 1 ist ausgebildet, in den Abgaskamin und/oder Rauchgaskamin und/oder Schornstein hineinzuragen. In einer Ausführungsform ragt das zweite Ende 1b des Gehäuses 1 in den Abgaskamin und/oder Rauchgaskamin und/oder Schornstein hinein.

In einer Ausführungsform umfasst das Gehäuse 1 zwischen dem ersten Ende 1a und dem zweiten Ende 2 einen rohrförmigen Abschnitt. Mithin ist das erste Ende 1a des Gehäuses 1 ein erstes Ende 1a des rohrförmigen Abschnittes. Das zweite Ende 1b des Gehäuses 1 ist ein zweites Ende 1b des rohrförmigen Abschnittes.

In einer Ausführungsform sind das erste Ende 1a des rohrförmigen Abschnittes und das zweite Ende 1b des rohrförmigen Abschnittes mindestens dreissig Millimeter voneinander beabstandet. Das erste Ende 1a und das zweite Ende 1b des rohrförmigen Abschnittes können mindestens sechzig Millimeter voneinander beabstandet sein. Das erste Ende 1a und das zweite Ende 1b des rohrförmigen Abschnittes können sogar mindestens einhundert Millimeter voneinander beabstandet sein.

Der rohrförmige Abschnitt kann beispielsweise eine runde Querschnittsform aufweisen. Der rohrförmige Abschnitt kann beispielsweise auch eine quadratische und/oder rechteckige Querschnittsform aufweisen.

Der rohrförmige Abschnitt kann beispielsweise eine Messspitze umfassen. Der rohrförmige Abschnitt kann insbesondere eine Messspitze sein. Mithin ist das erste Ende 1a des Gehäuses 1 ein erstes Ende 1a der Messspitze. Das zweite Ende 1b des Gehäuses 1 ist ein zweites Ende 1b der Messspitze.

In einer Ausführungsform sind das erste Ende 1a der Messspitze und das zweite Ende 1b der Messspitze mindestens dreissig Millimeter voneinander beabstandet. Das erste Ende 1a und das zweite Ende 1b der Messspitze können mindestens sechzig Millimeter voneinander beabstandet sein. Das erste Ende 1a und das zweite Ende 1b der Messspitze können sogar mindestens einhundert Millimeter voneinander beabstandet sein.

Die Messspitze kann beispielsweise eine runde Querschnittsform aufweisen. Die Messspitze kann beispielsweise auch eine quadratische und/oder rechteckige Querschnittsform aufweisen.

Das Gehäuse 1 weist eine erste Seite auf, welche sich zwischen dem ersten Ende 1a und dem zweiten Ende 1b des Gehäuses 1 erstreckt. Bei der ersten Seite des Gehäuses 1 kann es sich beispielsweise um eine Oberseite des Gehäuses 1 handeln. Ebenso kann es sich bei der ersten Seite des Gehäuses 1 um eine Unterseite des Gehäuses 1 handeln.

Insbesondere kann der rohrförmige Abschnitt eine erste Seite, welche sich zwischen dem ersten Ende 1a und dem zweiten Ende 1b des rohrförmigen Abschnittes erstreckt, aufweisen. Bei der ersten Seite des rohrförmigen Abschnittes kann es sich beispielsweise um eine Oberseite des rohrförmigen Abschnittes handeln. Ebenso kann es sich bei der ersten Seite des rohrförmigen Abschnittes um eine Unterseite des rohrförmigen Abschnittes handeln.

Ferner kann die Messspitze eine erste Seite, welche sich zwischen dem ersten Ende 1a und dem zweiten Ende 1b der Messspitze erstreckt, aufweisen. Bei der ersten Seite der Messspitze kann es sich beispielsweise um eine Oberseite der Messspitze handeln. Ebenso kann es sich bei der ersten Seite der Messspitze um eine Unterseite der Messspitze handeln.

Die erste Seite ist derart angeordnet, dass sie die Anströmung einer Vielzahl von Einlassöffnungen 4a - 4f durch Abgas und/oder Rauchgas ermöglicht. Entsprechend ist in FIG 1 eine Strömungsrichtung 5 in Richtung der ersten Seite des Gehäuses 1 eingezeichnet. Vorteilhaft ist in FIG 1 eine Strömungsrichtung 5 in Richtung der ersten Seite des rohrförmigen Abschnittes eingezeichnet. Idealerweise ist in FIG 1 eine Strömungsrichtung 5 in Richtung der ersten Seite der Messspitze eingezeichnet. Mithin ermöglicht die Strömungsrichtung 5 eine Anströmung der Vielzahl von Einlassöffnungen 4a - 4f. Insbesondere ermöglicht die Strömungsrichtung 5 eine Anströmung der Vielzahl von Einlassöffnungen 4a - 4f durch Abgas und/oder Rauchgas.

Entlang der ersten Seite ist die Vielzahl von Einlassöffnungen 4a - 4f angeordnet. Die Anordnung und/oder das Analysegerät kann beispielsweise zwei Einlassöffnungen entlang der ersten Seite umfassen. Die Anordnung und/oder das Analysegerät kann beispielsweise auch mehr zwei Einlassöffnungen entlang der ersten Seite umfassen. So kann die Anordnung und/oder das Analysegerät fünf Einlassöffnungen oder mehr als fünf Einlassöffnungen umfassen. Weiterhin kann die Anordnung und/oder das Analysegerät zehn Einlassöffnungen oder mehr als zehn Einlassöffnungen umfassen.

Ferner kann das Gehäuse 1 beispielsweise zwei Einlassöffnungen entlang der ersten Seite umfassen. Das Gehäuse 1 kann beispielsweise auch mehr zwei Einlassöffnungen entlang der ersten Seite umfassen. So kann das Gehäuse 1 fünf Einlassöffnungen oder mehr als fünf Einlassöffnungen umfassen. Weiterhin kann das Gehäuse 1 zehn Einlassöffnungen oder mehr als zehn Einlassöffnungen umfassen.

Ferner kann der rohrförmige Abschnitt beispielsweise zwei Einlassöffnungen entlang der ersten Seite umfassen. Der rohrförmige Abschnitt kann beispielsweise auch mehr zwei Einlassöffnungen entlang der ersten Seite umfassen. So kann der rohrförmige Abschnitt fünf Einlassöffnungen oder mehr als fünf Einlassöffnungen umfassen. Weiterhin kann der rohrförmige Abschnitt zehn Einlassöffnungen oder mehr als zehn Einlassöffnungen umfassen.

Darüber hinaus kann die Messspitze beispielsweise zwei Einlassöffnungen entlang der ersten Seite umfassen. Die Messspitze kann beispielsweise auch mehr zwei Einlassöffnungen entlang der ersten Seite umfassen. So kann die Messspitze fünf Einlassöffnungen oder mehr als fünf Einlassöffnungen umfassen. Weiterhin kann die Messspitze zehn Einlassöffnungen oder mehr als zehn Einlassöffnungen umfassen.

In einer Ausführungsform umfassen die einzelnen Einlassöffnungen der Vielzahl an Einlassöffnungen 4a - 4f jeweils eine Einlassöffnung für Abgas. In einer speziellen Ausführungsform sind die einzelnen Einlassöffnungen der Vielzahl an Einlassöffnungen 4a - 4f jeweils eine Einlassöffnung für Abgas. In einer weiteren Ausführungsform umfassen die einzelnen Einlassöffnungen der Vielzahl an Einlassöffnungen 4a - 4f jeweils eine Einlassöffnung für Rauchgas. In einer speziellen Ausführungsform sind die einzelnen Einlassöffnungen der Vielzahl an Einlassöffnungen 4a - 4f jeweils eine Einlassöffnung für Rauchgas.

Gemäss einem Aspekt der vorliegenden Offenbarung hat mindestens eine Einlassöffnung der Vielzahl an Einlassöffnungen 4a - 4f eine runde Querschnittsform. Gemäss einem weiteren Aspekt der vorliegenden Offenbarung hat mindestens eine Einlassöffnung der Vielzahl an Einlassöffnungen 4a - 4f eine quadratische und/oder rechteckige Querschnittsform.

Gemäss einem verwandten Aspekt der vorliegenden Offenbarung hat jede Einlassöffnung der Vielzahl an Einlassöffnungen 4a - 4f eine runde Querschnittsform. Gemäss einem weiteren, verwandten Aspekt der vorliegenden Offenbarung hat jede Einlassöffnung der Vielzahl an Einlassöffnungen 4a - 4f eine quadratische und/oder rechteckige Querschnittsform.

**Zur besseren Durchmischung** können die einzelnen Einlassöffnungen der Vielzahl an Einlassöffnungen 4a - 4f unterschiedliche Öffnungsquerschnitte aufweisen. So kann mindestens eine Einlassöffnung einen Öffnungsquerschnitt aufweisen, der verschieden ist von jedem Öffnungsquerschnitt der anderen Einlassöffnungen aus der Vielzahl an Einlassöffnungen 4a - 4f. Der Öffnungsquerschnitt der mindestens einen Einlassöffnung kann beispielsweise mindestens zehn Prozent verschieden sein vom Öffnungsquerschnitt der anderen Einlassöffnungen aus der Vielzahl an Einlassöffnungen 4a - 4f. Der Öffnungsquerschnitt der mindestens einen Einlassöffnung kann ebenso mindestens zwanzig Prozent verschieden sein vom Öffnungsquerschnitt der anderen Einlassöffnungen aus der Vielzahl an Einlassöffnungen 4a - 4f. Der Öffnungsquerschnitt der mindestens einen Einlassöffnung kann ebenso mindestens fünfzig Prozent verschieden sein vom Öffnungsquerschnitt der anderen Einlassöffnungen aus der Vielzahl an Einlassöffnungen 4a - 4f.

Das Gehäuse 1 weist eine zweite Seite auf, welche sich zwischen dem ersten Ende 1a und dem zweiten Ende 1b des Gehäuses 1 erstreckt. Die zweite Seite des Gehäuses 1 ist verschieden von der ersten Seite des Gehäuses 1. Mit anderen Worten, die erste und die zweite Seite überlappen nicht.

Die zweite Seite des Gehäuses 1 liegt idealerweise der ersten Seite des Gehäuses 1 gegenüber. Bei der zweiten Seite des Gehäuses 1 kann es sich beispielsweise um eine Unterseite des Gehäuses 1 handeln. Ebenso kann es sich bei der zweiten Seite des Gehäuses 1 um eine Oberseite des Gehäuses 1 handeln. In einer Ausführungsform ist die erste Seite des Gehäuses 1 parallel zur zweiten Seite des Gehäuses 1 angeordnet.

Insbesondere kann der rohrförmige Abschnitt eine zweite Seite, welche sich zwischen dem ersten Ende 1a und dem zweiten Ende 1b des rohrförmigen Abschnittes erstreckt, aufweisen. Die zweite Seite des rohrförmigen Abschnittes ist verschieden von der ersten Seite des rohrförmigen Abschnittes. Die zweite Seite des rohrförmigen Abschnittes liegt idealerweise der ersten Seite des rohrförmigen Abschnittes gegenüber. Bei der zweiten Seite des rohrförmigen Abschnittes kann es sich beispielsweise um eine Unterseite des rohrförmigen Abschnittes handeln. Ebenso kann es sich bei der zweiten Seite des rohrförmigen Abschnittes um eine Oberseite des rohrförmigen Abschnittes handeln. In einer Ausführungsform ist die erste Seite des rohrförmigen Abschnittes parallel zur zweiten Seite des rohrförmigen Abschnittes angeordnet.

Insbesondere kann die Messspitze eine zweite Seite, welche sich zwischen dem ersten Ende 1a und dem zweiten Ende 1b der Messspitze erstreckt, aufweisen. Die zweite Seite der Messspitze ist verschieden von der ersten Seite der Messspitze. Die zweite Seite der Messspitze liegt idealerweise der ersten Seite der Messspitze gegenüber. Bei der zweiten Seite der Messspitze kann es sich beispielsweise um eine Unterseite der Messspitze handeln. Ebenso kann es sich bei der zweiten Seite der Messspitze um eine Oberseite der Messspitze handeln. In einer Ausführungsform ist die erste Seite der Messspitze parallel zur zweiten Seite der Messspitze angeordnet.

Die zweite Seite ist der Ausführungsform gemäss FIG 1 derart angeordnet, dass sie die Ausströmung durch mindestens eine Auslassöffnung 6a ermöglicht. Entsprechend ist in FIG 1 eine Strömungsrichtung 7a weg von der zweiten Seite des Gehäuses 1 eingezeichnet. Vorteilhaft ist in FIG 1 eine Strömungsrichtung 7a weg von der zweiten Seite des rohrförmigen Abschnittes eingezeichnet. Idealerweise ist in FIG 1 eine Strömungsrichtung 7a weg von der zweiten Seite der Messspitze eingezeichnet. Mithin ermöglicht die Strömungsrichtung 7a eine Ausströmung durch die mindestens eine Auslassöffnung 6a. Insbesondere ermöglicht die Strömungsrichtung 7a eine Ausströmung von Abgas und/oder Rauchgas durch die mindestens eine Auslassöffnung 6a.

Die Platzierung der Sensoreinheit 8 und der Auslassöffnung 6a am zweiten Ende 1b wirkt sich günstig auf die Umströmung der Sensoreinheit 8 aus. Zudem ermöglicht die Platzierung der Auslassöffnung 6a am zweiten Ende 1b einige Flexibilität bei Montage der Auslassöffnung 6a in einem Abgaskamin und/oder Rauchgaskamin und/oder Schornstein.

Die Anordnung und/oder das Analysegerät umfasst demnach mindestens eine Auslassöffnung 6a. Die mindestens eine Auslassöffnung 6a ist näher am zweiten Ende 1b des Gehäuses 1 als am ersten Ende 1a des Gehäuses 1 angeordnet. Mit anderen Worten, es existiert ein erster Abstand $d_1$ der mindestens einen Auslassöffnung 6a vom ersten Ende 1a des Gehäuses 1. Es existiert zudem ein zweiter Abstand $d_2$ der mindestens einen Auslassöffnung 6a vom zweiten Ende 1b des Gehäuses 1. Dabei ist der erste Abstand $d_1$ grösser als der zweite Abstand $d_2$:

$$d_1 > d_2$$

In einer Ausführungsform ist die mindestens eine Auslassöffnung 6a näher am zweiten Ende 1b des rohrförmigen Abschnittes als am ersten Ende 1a des rohrförmigen Abschnittes angeordnet. Mit anderen Worten, es existiert ein erster Abstand $dr_1$ der mindestens einen Auslassöffnung 6a vom ersten Ende 1a des rohrförmigen Abschnittes. Es existiert zudem ein zweiter Abstand $dr_2$ der mindestens einen Auslassöffnung 6a vom zweiten Ende 1b des rohrförmigen Abschnittes. Dabei ist der erste Abstand $dr_1$ grösser als der zweite Abstand $dr_2$:

$$dr_1 > dr_2$$

Gemäss einer weiteren Ausführungsform ist die mindestens eine Auslassöffnung 6a näher am zweiten Ende 1b der Messspitze als am ersten Ende 1a der Messspitze angeordnet. Mit anderen Worten, es existiert ein erster Abstand $dm_1$ der mindestens einen Auslassöffnung 6a vom ersten Ende 1a der Messspitze. Es existiert zudem ein zweiter Abstand $dm_2$ der mindestens einen Auslassöffnung 6a vom zweiten Ende 1b der Messspitze. Dabei ist der erste Abstand $dm_1$ grösser als der zweite Abstand $dm_2$:

$$dm_1 > dm_2$$

Gemäss einem Aspekt der vorliegenden Offenbarung hat die mindestens eine Auslassöffnung 6a eine runde Querschnittsform. Gemäss einem weiteren Aspekt der vorliegenden Offenbarung hat die mindestens eine Auslassöffnung 6a eine quadratische und/oder rechteckige Querschnittsform.

In einer speziellen Ausführungsform weisen die mindestens eine Auslassöffnung 6a und jede der Einlassöffnungen der Vielzahl an Einlassöffnungen 4a - 4f die gleichen Querschnittsformen auf. Beispielsweise können die mindestens eine Auslassöffnung 6a und jede der Einlassöffnungen der Vielzahl an Einlassöffnungen 4a - 4f jeweils eine runde Querschnittsform aufweisen. Beispielsweise können die mindestens eine Auslassöffnung 6a und jede der Einlassöffnungen der Vielzahl an Einlassöffnungen 4a - 4f jeweils eine quadratische und/oder rechteckige Querschnittsform aufweisen. Gleiche Querschnittsformen der mindestens einen Auslassöffnung 6a und jeder der Einlassöffnungen der Vielzahl an Einlassöffnungen 4a - 4f verringern die Anzahl der bei der Herstellung benötigten Werkzeuge.

Ein hinreichend grosser Öffnungsquerschnitt der mindestens einen Auslassöffnung 6a hindert die Ausströmung des Abgases und/oder Rauchgases aus der mindestens einen Auslassöffnung 6a möglichst wenig. Der Öffnungsquerschnitt der mindestens einen Auslassöffnung 6a ist vorteilhaft mindestens so gross wie der kleinste Öffnungsquerschnitt unter der Vielzahl von Einlassöffnungen 4a - 4f. Der Öffnungsquerschnitt der mindestens einen Auslassöffnung 6a ist vorzugsweise mindestens so gross wie das arithmetische Mittel der Öffnungsquerschnitte der Vielzahl von Einlassöffnungen 4a - 4f. Der Öffnungsquerschnitt der mindestens einen Auslassöffnung 6a ist idealerweise mindestens so gross wie der grösste Öffnungsquerschnitt unter der Vielzahl von Einlassöffnungen 4a - 4f. Der Öffnungsquerschnitt der mindestens einen Auslassöffnung 6a kann sogar grösser als der grösste Öffnungsquerschnitt unter der Vielzahl von Einlassöffnungen 4a - 4f sein.

Die Anordnung und/oder das Analysegerät umfasst weiterhin eine Sensoreinheit 8. Die Sensoreinheit 8 ist in dem Gehäuse 1 angeordnet. Die Sensoreinheit 1 ist nahe dem zweiten Ende 1b des Gehäuses 1 angeordnet. Das heisst, dass die Sensoreinheit 8 näher am zweiten Ende 1b des Gehäuses 1 angeordnet ist als am ersten Ende des Gehäuses 1. Mit anderen Worten, es existiert ein erster Abstand der Sensoreinheit 8 vom ersten Ende 1a des Gehäuses 1. Es existiert zudem ein zweiter Abstand der Sensoreinheit 8 vom zweiten Ende 1b des Gehäuses 1. Dabei ist der erste Abstand grösser als der zweite Abstand.

Ferner kann die Sensoreinheit 8 in dem rohrförmigen Abschnitt angeordnet und befestigt sein. Die Sensoreinheit 8 ist nahe dem zweiten Ende 1b des rohrförmigen Abschnittes angeordnet. Das heisst, dass die Sensoreinheit 8 näher am zweiten Ende 1b des rohrförmigen Abschnittes angeordnet ist als am ersten Ende 1a des rohrförmigen Abschnittes. Mit anderen Worten, es existiert ein erster Abstand der Sensoreinheit 8 vom ersten Ende 1a des rohrförmigen Abschnittes. Es existiert zudem ein zweiter Abstand der Sensoreinheit 8 vom zweiten Ende 1b des rohrförmigen Abschnittes. Dabei ist der erste Abstand grösser als der zweite Abstand.

Darüber hinaus kann die Sensoreinheit 8 in der Messspitze angeordnet und befestigt sein. Die Sensoreinheit 8 ist nahe dem zweiten Ende 1b der Messspitze angeordnet. Das heisst, dass die Sensoreinheit 8 näher am zweiten Ende 1b der Messspitze angeordnet ist als am ersten Ende 1a der Messspitze. Mit anderen Worten, es existiert ein erster Abstand der Sensoreinheit 8 vom ersten Ende 1a der Messspitze. Es existiert zudem ein zweiter Abstand der Sensoreinheit 8 vom zweiten Ende 1b der Messspitze. Dabei ist der erste Abstand grösser als der zweite Abstand.

Die Sensoreinheit 8 ist vorzugsweise zwischen der Vielzahl an Einlassöffnungen 4a - 4f und der mindestens einen Auslassöffnung 6a angeordnet. Die Anordnung ermöglicht eine Strömung von der Vielzahl an Einlassöffnungen 4a - 4f hin zur Sensoreinheit 8. Die Anordnung ermöglicht ferner eine fortgesetzte Strömung von der **Sensoreinheit 8** zur mindestens einen Auslassöffnung 6a. Die Anordnung ermöglicht insbesondere eine Strömung von Abgas und/oder Rauchgas von der Vielzahl an Einlassöffnungen 4a - 4f hin zur Sensoreinheit 8. Die Anordnung ermöglicht ferner eine fortgesetzte Strömung von Abgas und/oder Rauchgas von der **Sensoreinheit 8** zur mindestens einen Auslassöffnung 6a.

Im Hinblick auf die Mittelung der Beiträge der Einlassöffnungen aus der Vielzahl von Einlassöffnungen 4a - 4f ist die Sensoreinheit 8 nahe der mindestens einen Auslassöffnung 6a angeordnet. Beispielsweise kann ein kürzester Abstand zwischen der Sensoreinheit 8 und der Auslassöffnung 6a weniger als fünfundzwanzig Millimeter betragen. Insbesondere kann ein kürzester Abstand zwischen der Sensoreinheit 8 und der Auslassöffnung 6a weniger als zwölf Millimeter betragen. Ein kürzester Abstand zwischen der Sensoreinheit 8 und der Auslassöffnung 6a kann sogar weniger als sechs Millimeter betragen.

Gemäss einem Aspekt der vorliegenden Offenbarung kann die Sensoreinheit 8 einen Sensor basierend auf Zirconiumdioxid umfassen.

Die Sensoreinheit 8 kann beispielsweise über ein Paar elektrische Leitungen 9a, 9b mit einer Auswerteeinheit verbunden werden. Das Paar elektrischer Leitungen 9a, 9b kann sich beispielsweise von der Sensoreinheit 8 zum Flansch 3 erstrecken. Das Paar elektrischer Leitungen 9a, 9b ist vorzugsweise so elektrisch isoliert, dass die jeweiligen elektrischen Isolierungen den Temperaturen in einem Abgaskamin und/oder Rauchgaskamin und/oder Schornstein standhält. Insbesondere kann das Paar elektrischer Leitungen 9a, 9b derart isoliert sein, dass es Temperaturen von 363 Kelvin standhält. Vorzugsweise kann das Paar elektrischer Leitungen 9a, 9b derart isoliert sein, dass es Temperaturen von 378 Kelvin standhält. Idealerweise kann das Paar elektrischer Leitungen 9a, 9b derart isoliert sein, dass es Temperaturen von 393 Kelvin standhält. Eine temperaturbeständige elektrische Isolierung führt zu einer hitzebeständigen Anordnung und/oder zu einem hitzebeständigen Analysegerät.

In einer Ausführungsform umfasst mindestens eine elektrische Leitung des Paares elektrischer Leitungen 9a, 9b zugleich eine mechanische Befestigung der Sensoreinheit 8. Beispielsweise kann mindestens eine elektrische Leitung des Paares elektrischer Leitungen 9a, 9b zugleich eine mechanische Befestigung der Sensoreinheit 8 gegenüber dem Gehäuse 1 umfassen. Ferner kann mindestens eine elektrische Leitung des Paares elektrischer Leitungen 9a, 9b zugleich eine mechanische Befestigung der Sensoreinheit 8 gegenüber dem rohrförmigen Abschnitt umfassen. Darüber hinaus kann mindestens eine elektrische Leitung des Paares elektrischer Leitungen 9a, 9b zugleich eine mechanische Befestigung der Sensoreinheit 8 gegenüber der Messspitze umfassen.

In einer besonderen Ausführungsform umfasst mindestens eine elektrische Leitung des Paares elektrischer Leitungen 9a, 9b zugleich eine ausschliessliche mechanische Befestigung der Sensoreinheit 8. Beispielsweise kann mindestens eine elektrische Leitung des Paares elektrischer Leitungen 9a, 9b zugleich eine ausschliessliche mechanische Befestigung der Sensoreinheit 8 gegenüber dem Gehäuse 1 umfassen. Ferner kann mindestens eine elektrische Leitung des Paares elektrischer Leitungen 9a, 9b zugleich eine ausschliessliche mechanische Befestigung der Sensoreinheit 8 gegenüber dem rohrförmigen Abschnitt umfassen. Darüber hinaus kann mindestens eine elektrische Leitung des Paares elektrischer Leitungen 9a, 9b zugleich eine ausschliessliche mechanische Befestigung der Sensoreinheit 8 gegenüber der Messspitze umfassen. Eine ausschliessliche mechanische Befestigung stützt die Sensoreinheit 8 derart, dass für eine stabile Anordnung der Sensoreinheit 8 keine weitere mechanische Befestigung erforderlich ist.

Indem mindestens eine elektrische Leitung des Paares elektrischer Leitungen 9a, 9b zugleich als mechanische Befestigung dient, verringert sich die Anzahl benötigter Teile der Anordnung und/oder des Analysegerätes. Infolge der geringeren Anzahl benötigter Teile können weniger solche Teile ausfallen und/oder versagen. Mithin die Anordnung und/oder das Analysegerät robuster.

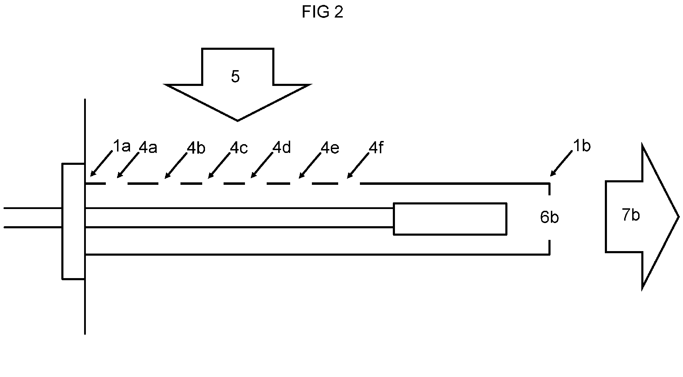

Gemäss dem in FIG 2 gezeigten Beispiel kann die mindestens eine Auslassöffnung 6b auch weder an der ersten noch an der zweiten Seite angeordnet sein. Insbesondere ist die mindestens eine seitwärts gerichtete Auslassöffnung 6b weder an der ersten noch an der zweiten Seite des Gehäuses 1 angeordnet. Ferner ist die mindestens eine seitwärts gerichtete Auslassöffnung 6b weder an der ersten noch an der zweiten Seite des rohrförmigen Abschnittes angeordnet. Darüber hinaus ist die mindestens eine seitwärts gerichtete Auslassöffnung 6b weder an der ersten noch an der zweiten Seite der Messspitze angeordnet.

Stattdessen ist die mindestens eine seitwärts gerichtete Auslassöffnung 6b seitlich am zweiten Ende 1b des Gehäuses 1 angeordnet. Mithin umfasst das Gehäuse 1 die mindestens eine seitwärts gerichtete Auslassöffnung 6b. Ferner kann die mindestens eine seitwärts gerichtete Auslassöffnung 6b seitlich am zweiten Ende 1b des rohrförmigen Abschnittes angeordnet sein. Mithin umfasst der rohrförmige Abschnitt die mindestens eine seitwärts gerichtete Auslassöffnung 6b. Darüber hinaus kann die mindestens eine seitwärts gerichtete Auslassöffnung 6b seitlich am zweiten Ende 1b der Messspitze angeordnet sein. Mithin umfasst die Messspitze die mindestens eine seitwärts gerichtete Auslassöffnung 6b.

Entsprechend ist in FIG 2 eine seitwärts gerichtete Strömungsrichtung 7b weg vom zweiten Ende 1b des Gehäuses 1 eingezeichnet. Vorteilhaft ist in FIG 1 eine seitwärts gerichtete Strömungsrichtung 7b weg vom zweiten Ende 1b des rohrförmigen Abschnittes eingezeichnet. Idealerweise ist in FIG 1 eine seitwärts gerichtete Strömungsrichtung 7b weg vom zweiten Ende 1b der Messspitze eingezeichnet. Mithin ermöglicht die seitwärts gerichtete Strömungsrichtung 7b eine Ausströmung durch die mindestens eine seitwärts gerichtete Auslassöffnung 6b. Insbesondere ermöglicht die seitwärts gerichtete Strömungsrichtung 7b eine Ausströmung von Abgas und/oder Rauchgas durch die mindestens eine seitwärts gerichtete Auslassöffnung 6b.

Die mindestens eine seitwärts gerichtete Auslassöffnung 6b ist unmittelbar am zweiten Ende 1b des Gehäuses 1 angeordnet. Mit anderen Worten, es existiert ein erster Abstand der mindestens einen seitwärts gerichteten Auslassöffnung 6b vom ersten Ende 1a des Gehäuses 1. Es existiert zudem ein zweiter Abstand der mindestens einen seitwärts gerichteten Auslassöffnung 6b vom zweiten Ende 1b des Gehäuses 1. Dabei ist der erste Abstand grösser als der zweite Abstand.

In einer Ausführungsform ist die mindestens eine seitwärts gerichteten Auslassöffnung 6b unmittelbar am zweiten Ende 1b des rohrförmigen Abschnittes angeordnet. Mit anderen Worten, es existiert ein erster Abstand der mindestens einen seitwärts gerichteten Auslassöffnung 6b vom ersten Ende 1a des rohrförmigen Abschnittes. Es existiert zudem ein zweiter Abstand der mindestens einen seitwärts gerichteten Auslassöffnung 6b vom zweiten Ende 1b des rohrförmigen Abschnittes. Dabei ist der erste Abstand grösser als der zweite Abstand.

Gemäss einer weiteren Ausführungsform ist die mindestens eine seitwärts gerichteten Auslassöffnung 6b unmittelbar am zweiten Ende 1b der Messspitze angeordnet. Mit anderen Worten, es existiert ein erster Abstand der mindestens einen seitwärts gerichteten Auslassöffnung 6b vom ersten Ende 1a der Messspitze. Es existiert zudem ein zweiter Abstand der mindestens einen seitwärts gerichteten Auslassöffnung 6b vom zweiten Ende 1b der Messspitze. Dabei ist der erste Abstand grösser als der zweite Abstand.

Gemäss einem Aspekt des vorliegenden Beispiels hat die mindestens eine seitwärts gerichtete Auslassöffnung 6b eine runde Querschnittsform. Gemäss einem weiteren Aspekt der vorliegenden Offenbarung hat die mindestens eine seitwärts gerichtete Auslassöffnung 6b eine quadratische und/oder rechteckige Querschnittsform.

In einer speziellen Ausführungsform weisen die mindestens eine seitwärts gerichtete Auslassöffnung 6b und jede der Einlassöffnungen der Vielzahl an Einlassöffnungen 4a - 4f die gleichen Querschnittsformen auf. Beispielsweise können die mindestens eine seitwärts gerichtete Auslassöffnung 6b und jede der Einlassöffnungen der Vielzahl an Einlassöffnungen 4a - 4f jeweils eine runde Querschnittsform aufweisen. Beispielsweise können die mindestens eine seitwärts gerichtete Auslassöffnung 6b und jede der Einlassöffnungen der Vielzahl an Einlassöffnungen 4a - 4f jeweils eine quadratische und/oder rechteckige Querschnittsform aufweisen. Gleiche Querschnittsformen der mindestens einen Auslassöffnung 6b und jeder der Einlassöffnungen der Vielzahl an Einlassöffnungen 4a - 4f verringern die Anzahl der bei der Herstellung benötigten Werkzeuge.

Ein hinreichend grosser Öffnungsquerschnitt der mindestens einen seitwärts gerichteten Auslassöffnung 6b hindert die Ausströmung des Abgases und/oder Rauchgases aus der mindestens einen Auslassöffnung 6b möglichst wenig. Der Öffnungsquerschnitt der mindestens einen seitwärts gerichteten Auslassöffnung 6b ist vorteilhaft mindestens so gross wie der kleinste Öffnungsquerschnitt unter der Vielzahl von Einlassöffnungen 4a - 4f. Der Öffnungsquerschnitt der mindestens einen seitwärts gerichteten Auslassöffnung 6b ist vorzugsweise mindestens so gross wie das arithmetische Mittel der Öffnungsquerschnitte der Vielzahl von Einlassöffnungen 4a - 4f. Der Öffnungsquerschnitt der mindestens einen seitwärts gerichteten Auslassöffnung 6b ist idealerweise mindestens so gross wie der grösste Öffnungsquerschnitt unter der Vielzahl von Einlassöffnungen 4a - 4f. Der Öffnungsquerschnitt der mindestens einen seitwärts gerichteten Auslassöffnung 6b kann sogar grösser als der grösste Öffnungsquerschnitt unter der Vielzahl von Einlassöffnungen 4a - 4f sein.

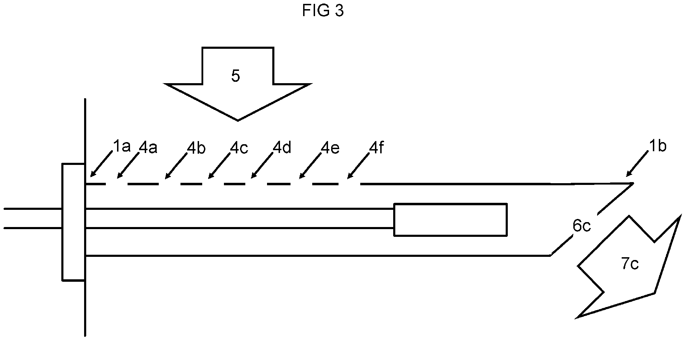

Gemäss dem in FIG 3 gezeigten Beispiel kann die mindestens eine Auslassöffnung 6c auch weder an der ersten noch an der zweiten Seite angeordnet sein. Insbesondere ist die mindestens eine schräge Auslassöffnung 6c weder an der ersten noch an der zweiten Seite des Gehäuses 1 angeordnet. Ferner ist die mindestens eine schräge Auslassöffnung 6c weder an der ersten noch an der zweiten Seite des rohrförmigen Abschnittes angeordnet.

Darüber hinaus ist die mindestens eine schräge Auslassöffnung 6c weder an der ersten noch an der zweiten Seite der Messspitze angeordnet.

Stattdessen ist die mindestens eine schräge Auslassöffnung 6c an einem abgeschrägten Ende 1b des Gehäuses 1 angeordnet. Mithin umfasst das Gehäuse 1 die mindestens eine schräge Auslassöffnung 6c. Idealerweise umfasst das abgeschrägte Ende 1b des Gehäuses 1 die mindestens eine schräge Auslassöffnung 6c. Ferner kann die mindestens eine schräge Auslassöffnung 6c an einem abgeschrägten Ende 1b des rohrförmigen Abschnittes angeordnet sein. Mithin umfasst der rohrförmige Abschnitt die mindestens eine schräge Auslassöffnung 6c. Idealerweise umfasst das abgeschrägte Ende 1b des rohrförmigen Abschnittes die mindestens eine schräge Auslassöffnung 6c. Darüber hinaus kann die mindestens eine schräge Auslassöffnung 6c an einem abgeschrägten Ende 1b der Messspitze angeordnet sein. Mithin umfasst die Messspitze die mindestens eine schräge Auslassöffnung 6c. Idealerweise umfasst das abgeschrägte Ende 1b der Messspitze die mindestens eine schräge Auslassöffnung 6c.

Entsprechend ist in FIG 3 eine schräg gerichtete Strömungsrichtung 7c weg vom zweiten Ende 1b des Gehäuses 1 eingezeichnet. Vorteilhaft ist in FIG 1 eine schräg gerichtete Strömungsrichtung 7c weg vom zweiten Ende 1b des rohrförmigen Abschnittes eingezeichnet. Idealerweise ist in FIG 1 eine schräg gerichtete Strömungsrichtung 7c weg vom zweiten Ende 1b der Messspitze eingezeichnet. Mithin ermöglicht die schräg gerichtete Strömungsrichtung 7c eine Ausströmung durch die mindestens eine schräge Auslassöffnung 6c. Insbesondere ermöglicht die schräg gerichtete Strömungsrichtung 7c eine Ausströmung von Abgas und/oder Rauchgas durch die mindestens eine schräge Auslassöffnung 6c.

Die mindestens eine schräge Auslassöffnung 6c ist am zweiten Ende 1b des Gehäuses 1 angeordnet. Mit anderen Worten, es existiert ein erster Abstand der mindestens einen schrägen Auslassöffnung 6c vom ersten Ende 1a des Gehäuses 1. Es existiert zudem ein zweiter Abstand der mindestens einen schrägen Auslassöffnung 6c vom zweiten Ende 1b des Gehäuses 1. Dabei ist der erste Abstand grösser als der zweite Abstand.

In einer Ausführungsform ist die mindestens eine schräge Auslassöffnung 6c am zweiten Ende 1b des rohrförmigen Abschnittes angeordnet. Mit anderen Worten, es existiert ein erster Abstand der mindestens einen schrägen Auslassöffnung 6c vom ersten Ende 1a des rohrförmigen Abschnittes. Es existiert zudem ein zweiter Abstand der mindestens einen schrägen Auslassöffnung 6c vom zweiten Ende 1b des rohrförmigen Abschnittes. Dabei ist der erste Abstand grösser als der zweite Abstand.

Gemäss einer weiteren Ausführungsform ist die mindestens eine schräge Auslassöffnung 6c am zweiten Ende 1b der Messspitze angeordnet. Mit anderen Worten, es existiert ein erster Abstand der mindestens einen schrägen Auslassöffnung 6c vom ersten Ende 1a der Messspitze. Es existiert zudem ein zweiter Abstand der mindestens einen schrägen Auslassöffnung 6c vom zweiten Ende 1b der Messspitze. Dabei ist der erste Abstand grösser als der zweite Abstand.

Gemäss einem Aspekt des vorliegenden Beispiels hat die mindestens eine schräge Auslassöffnung 6c eine runde Querschnittsform. Gemäss einem weiteren Aspekt der vorliegenden Offenbarung hat die mindestens eine schräge Auslassöffnung 6c eine quadratische und/oder rechteckige Querschnittsform.

In einer speziellen Ausführungsform weisen die mindestens eine schräge Auslassöffnung 6c und jede der Einlassöffnungen der Vielzahl an Einlassöffnungen 4a - 4f die gleichen Querschnittsformen auf. Beispielsweise können die mindestens eine schräge Auslassöffnung 6c und jede der Einlassöffnungen der Vielzahl an Einlassöffnungen 4a - 4f jeweils eine runde Querschnittsform aufweisen. Beispielsweise können die mindestens eine schräge Auslassöffnung 6c und jede der Einlassöffnungen der Vielzahl an Einlassöffnungen 4a - 4f jeweils eine quadratische und/oder rechteckige Querschnittsform aufweisen. Gleiche Querschnittsformen der mindestens einen schrägen Auslassöffnung 6c und jeder der Einlassöffnungen der Vielzahl an Einlassöffnungen 4a - 4f verringern die Anzahl der bei der Herstellung benötigten Werkzeuge.

Ein hinreichend grosser Öffnungsquerschnitt der mindestens einen schrägen Auslassöffnung 6c hindert die Ausströmung des Abgases und/oder Rauchgases aus der mindestens einen schrägen Auslassöffnung 6c möglichst wenig. Der Öffnungsquerschnitt der mindestens einen schrägen Auslassöffnung 6c ist vorteilhaft mindestens so gross wie der kleinste Öffnungsquerschnitt unter der Vielzahl von Einlassöffnungen 4a - 4f. Der Öffnungsquerschnitt der mindestens einen schrägen Auslassöffnung 6c ist vorzugsweise mindestens so gross wie das arithmetische Mittel der Öffnungsquerschnitte der Vielzahl von Einlassöffnungen 4a - 4f. Der Öffnungsquerschnitt der mindestens einen schrägen Auslassöffnung 6c ist idealerweise mindestens so gross wie der grösste Öffnungsquerschnitt unter der Vielzahl von Einlassöffnungen 4a - 4f. Der Öffnungsquerschnitt der mindestens einen schrägen Auslassöffnung 6c kann sogar grösser als der grösste Öffnungsquerschnitt unter der Vielzahl von Einlassöffnungen 4a - 4f sein.

Mit anderen Worten, die vorliegende Offenbarung bezieht sich auf eine Anordnung umfassend eine Sensoreinheit (8) und ein Gehäuse (1) mit einem ersten (1a) und einem zweiten Ende (1b), mit einer Vielzahl an Einlassöffnungen (4a - 4f) und mit mindestens einer Auslassöffnung (6a - 6c);

> wobei die Anordnung am ersten Ende (1a) des Gehäuses (1) eine Befestigungsvorrichtung (3) zur mechanischen Befestigung des Gehäuses (1) an einer Wand (2) umfasst, sodass das Gehäuse (1) gegenüber der Wand (2) im Wesentlichen unbeweglich ist;
> wobei das erste Ende (1a) verschieden ist vom zweiten Ende (1b) und das erste Ende (1a) dem zweiten Ende (1b) gegenüberliegt und die mindestens eine Auslassöffnung (6a - 6c) verschieden ist von jeder Einlassöffnung der Vielzahl an Einlassöffnungen (4a - 4f);
> wobei die Sensoreinheit (8) innen im Gehäuse (1) angeordnet ist und einen ersten Abstand vom ersten Ende (1a) und einen zweiten Abstand vom zweiten Ende aufweist;
> wobei der erste Abstand der Sensoreinheit (8) vom ersten Ende (1a) grösser ist als der zweite Abstand der Sensoreinheit (8) vom zweiten Ende (1b);
> wobei die mindestens eine Auslassöffnung (6a - 6c) einen ersten Abstand vom ersten Ende (1a) und einen zweiten Abstand vom zweiten Ende (1b) aufweist; und
> wobei der erste Abstand der mindestens einen Auslassöffnung (6a - 6c) vom ersten Ende (1a) grösser ist als der zweite Abstand der mindestens einen Auslassöffnung (6a - 6c) vom zweiten Ende (1b).

In einer Ausführungsform umfasst die Anordnung am ersten Ende (1a) des Gehäuses (1) die Befestigungsvorrichtung (3) zur mechanischen Befestigung des Gehäuses (1) an einer Wand (2), sodass das Gehäuse (1) gegenüber der Wand (2) unbeweglich ist.

In einer Ausführungsform ist das zweite Ende (1b) des Gehäuses (1) ein freies Ende des Gehäuses (1). Insbesondere ist das zweite Ende (1b) des Gehäuses (1) nicht ausgebildet zur Befestigung an einer Wand (2). Das zweite Ende (1b) des Gehäuses (1) zeigt vorteilhaft in einen Abgaskamin und/oder Rauchgaskamin und/oder Schornstein hinein oder ist dazu ausgebildet.

Vorzugsweise ist mindestens eine Einlassöffnung der Vielzahl an Einlassöffnungen (4a - 4f) ausgebildet als Einlassöffnung für Abgas und/oder Rauchgas in das Gehäuse (1). Idealerweise ist jede Einlassöffnung der Vielzahl an Einlassöffnungen (4a - 4f) ausgebildet als Einlassöffnung für Abgas und/oder Rauchgas in das Gehäuse (1).

Vorzugsweise ist die mindestens eine Auslassöffnung (6a - 6c) ausgebildet als Auslassöffnung für Abgas und/oder Rauchgas aus dem Gehäuse (1).

Die vorliegende Offenbarung lehrt auch eine der vorgenannten Anordnungen, wobei Sensoreinheit (8) in Fluidverbindung mit jeder Einlassöffnung der Vielzahl an Einlassöffnungen (4a - 4f) und mit der mindestens einen Auslassöffnung (6a - 6c) ist.

Gemäss einem Aspekt der vorliegenden Offenbarung umfasst die Anordnung ein Analysegerät. Gemäss einem speziellen Aspekt der vorliegenden Offenbarung ist die Anordnung ein Analysegerät.

Gemäss einem Aspekt der vorliegenden Offenbarung umfasst die Anordnung eine Anordnung zur Analyse von Abgasen und/oder Rauchgasen. Gemäss einem speziellen Aspekt der vorliegenden Offenbarung ist die Anordnung eine Anordnung zur Analyse von Abgasen und/oder Rauchgasen.

Gemäss einem Aspekt der vorliegenden Offenbarung umfasst die Anordnung ein Analysegerät zur Analyse von Abgasen und/oder Rauchgasen. Gemäss einem speziellen Aspekt der vorliegenden Offenbarung ist die Anordnung ein Analysegerät zur Analyse von Abgasen und/oder Rauchgasen.

Die vorliegende Offenbarung lehrt ferner eine der vorgenannten Anordnungen,

> wobei die Befestigungsvorrichtung (3) einen ersten Abstand vom ersten Ende (1a) und einen zweiten Abstand vom zweiten Ende aufweist; und
> wobei der erste Abstand der Befestigungsvorrichtung (3) vom ersten Ende (1a) kleiner ist als der zweite Abstand der Befestigungsvorrichtung (3) vom zweiten Ende (1b).

Die vorliegende Offenbarung lehrt ebenfalls eine der vorgenannten Anordnungen, wobei die Befestigungsvorrichtung (3) einen Flansch umfasst.

Die vorliegende Offenbarung lehrt weiterhin eine der vorgenannten Anordnungen, wobei die Befestigungsvorrichtung (3) ein Flansch ist. Die vorliegende Offenbarung lehrt zudem eine der vorgenannten Anordnungen, wobei die Befestigungsvorrichtung (3) eine Verschraubung umfasst. Die vorliegende Offenbarung lehrt darüber hinaus eine der vorgenannten Anordnungen, wobei die Befestigungsvorrichtung (3) eine Verschraubung ist. Die vorliegende Offenbarung lehrt ferner eine der vorgenannten Anordnungen, wobei die Befestigungsvorrichtung (3) eine Steckverbindung umfasst. Die vorliegende Offenbarung lehrt ausserdem eine der vorgenannten Anordnungen, wobei die Befestigungsvorrichtung (3) eine Steckverbindung ist.

Die vorliegende Offenbarung lehrt zudem eine der vorgenannten Anordnungen,

wobei die Anordnung ein Paar elektrischer Leitungen (9a, 9b) umfasst; und
wobei sich mindestens eine elektrische Leitung des Paares elektrischer Leitungen (9a, 9b) von der Sensoreinheit (8) zur Befestigungsvorrichtung (3) erstreckt.

Die vorliegende Offenbarung lehrt weiterhin eine der vorgenannten Anordnungen,

wobei die Anordnung ein Paar elektrischer Leitungen (9a, 9b) umfasst; und
wobei sich jede elektrische Leitung des Paares elektrischer Leitungen (9a, 9b) von der Sensoreinheit (8) zur Befestigungsvorrichtung (3) erstreckt.

Vorzugsweise verläuft das Paar elektrischer Leitungen (9a, 9b) innerhalb des Gehäuses (1).

Die vorliegende Offenbarung lehrt ebenfalls eine der vorgenannten Anordnungen umfassend ein Paar elektrischer Leitungen (9a, 9b), wobei mindestens eine elektrische Leitung des Paares elektrischer Leitungen (9a, 9b) die Sensoreinheit (8) gegenüber dem Gehäuse (1) mechanisch stützt.

Die vorliegende Offenbarung lehrt weiterhin eine der vorgenannten Anordnungen umfassend ein Paar elektrischer Leitungen (9a, 9b),

wobei die Befestigungsvorrichtung (3) an dem Gehäuse (1) mechanisch befestigt ist; und
wobei mindestens eine elektrische Leitung des Paares elektrischer Leitungen (9a, 9b) an der Befestigungsvorrichtung (3) mechanisch befestigt ist, sodass die mindestens eine elektrische Leitung des Paares elektrischer Leitungen (9a, 9b) die Sensoreinheit (8) gegenüber dem Gehäuse (1) mechanisch stützt.

Die vorliegende Offenbarung lehrt zudem eine der vorgenannten Anordnungen umfassend ein Paar elektrischer Leitungen (9a, 9b),

wobei die Befestigungsvorrichtung (3) an dem Gehäuse (1) mechanisch befestigt ist; und
wobei ausschliesslich eine elektrische Leitung des Paares elektrischer Leitungen (9a, 9b) an der Befestigungsvorrichtung (3) mechanisch befestigt ist, sodass die ausschliesslich eine an der Befestigungsvorrichtung (3) befestigte elektrische Leitung des Paares elektrischer Leitungen (9a, 9b) die Sensoreinheit (8) gegenüber dem Gehäuse (1) ausschliesslich mechanisch stützt.

Die vorliegende Offenbarung lehrt darüber hinaus eine der vorgenannten Anordnungen umfassend ein Paar elektrischer Leitungen (9a, 9b), wobei mindestens eine weitere elektrische Leitung des Paares elektrischer Leitungen (9a, 9b) direkt an dem Gehäuse (1) mechanisch befestigt ist, sodass die mindestens eine weitere elektrische Leitung des Paares elektrischer Leitungen (9a, 9b) die Sensoreinheit (8) gegenüber dem Gehäuse (1) mechanisch stützt.

Die vorliegende Offenbarung lehrt weiterhin eine der vorgenannten Anordnungen umfassend ein Paar elektrischer Leitungen (9a, 9b), wobei ausschliesslich eine weitere elektrische Leitung des Paares elektrischer Leitungen (9a, 9b) direkt an dem Gehäuse (1) mechanisch befestigt ist, sodass die ausschliesslich eine weitere elektrische Leitung des Paares elektrischer Leitungen (9a, 9b) die Sensoreinheit (8) gegenüber dem Gehäuse (1) ausschliesslich mechanisch stützt.

Die vorliegende Offenbarung lehrt ferner eine der vorgenannten Anordnungen umfassend ein Paar elektrischer Leitungen (9a, 9b),

wobei die Anordnung eine Auswerteeinheit umfasst; und
wobei mindestens eine elektrische Leitung des Paares elektrischer Leitungen (9a, 9b) die Sensoreinheit (8) mit der Auswerteeinheit elektrisch verbindet.

Die vorliegende Offenbarung lehrt weiterhin eine der vorgenannten Anordnungen umfassend ein Paar elektrischer Leitungen (9a, 9b),

wobei die Anordnung eine Auswerteeinheit umfasst; und
wobei mindestens eine elektrische Leitung des Paares elektrischer Leitungen (9a, 9b) die Sensoreinheit (8) mit der Auswerteeinheit galvanisch verbindet.

Die vorliegende Offenbarung lehrt zudem eine der vorgenannten Anordnungen, wobei die Anordnung genau eine Auslassöffnung (6a - 6c) umfasst.

Die vorliegende Offenbarung lehrt darüber hinaus eine der vorgenannten Anordnungen,

wobei das Gehäuse (1) eine erste Seite umfasst, welche sich vom ersten Ende (1a) des Gehäuses (1) zum zweiten Ende (1b) des Gehäuses (1) erstreckt;
wobei das Gehäuse (1) eine zweite Seite umfasst, welche sich vom ersten Ende (1a) des Gehäuses (1) zum zweiten Ende (1b) des Gehäuses (1) erstreckt;
wobei die zweite Seite verschieden ist von der ersten Seite;
wobei mindestens eine Einlassöffnung der Vielzahl an Einlassöffnungen (4a - 4f) an der ersten Seite angeordnet ist; und
wobei die mindestens eine Auslassöffnung (6a) an der zweiten Seite angeordnet ist.

Die vorliegende Offenbarung lehrt ausserdem eine der vorgenannten Anordnungen umfassend eine erste Seite,
wobei jede Einlassöffnung der Vielzahl an Einlassöffnungen (4a - 4f) an der ersten Seite angeordnet ist.

Die vorliegende Offenbarung lehrt ferner eine der vorgenannten Anordnungen umfassend eine erste und eine zweite Seite, wobei die zweite Seite parallel zur ersten Seite verläuft.

Die vorliegende Offenbarung lehrt zudem eine der vorgenannten Anordnungen,
wobei die mindestens eine Auslassöffnung (6b) unmittelbar am zweiten Ende (1b) angeordnet ist.

Die vorliegende Offenbarung lehrt ausserdem eine der vorgenannten Anordnungen umfassend einen rohrförmigen Abschnitt,

wobei das Gehäuse (1) eine erste Seite umfasst, welche sich vom ersten Ende (1a) des Gehäuses (1) zum zweiten Ende (1b) des Gehäuses (1) erstreckt;
wobei das Gehäuse (1) eine zweite Seite umfasst, welche sich vom ersten Ende (1a) des Gehäuses (1) zum zweiten Ende (1b) des Gehäuses (1) erstreckt;
wobei die zweite Seite verschieden ist von der ersten Seite;
wobei mindestens eine Einlassöffnung der Vielzahl an Einlassöffnungen (4a - 4f) an der ersten Seite angeordnet ist; und
wobei die mindestens eine Auslassöffnung (6b) ausserhalb der ersten Seite und ausserhalb der zweiten Seite angeordnet ist.

Die vorliegende Offenbarung lehrt ferner eine der vorgenannten Anordnungen umfassend einen rohrförmigen Abschnitt,

wobei das Gehäuse (1) an seinem zweiten Ende (1b) einen rohrförmigen Abschnitt umfasst; und
wobei die mindestens eine Auslassöffnung (6b) ein offenes Ende des rohrförmigen Abschnittes umfasst.

Die vorliegende Offenbarung lehrt zudem eine der vorgenannten Anordnungen umfassend einen rohrförmigen Abschnitt,
wobei die mindestens eine Auslassöffnung (6b) ein offenes Ende des rohrförmigen Abschnittes ist.

Die vorliegende Offenbarung lehrt weiterhin eine der vorgenannten Anordnungen umfassend einen rohrförmigen Abschnitt,
wobei die mindestens eine Auslassöffnung (6b) ein vollständig geöffnetes Ende des rohrförmigen Abschnittes umfasst.

Die vorliegende Offenbarung lehrt zudem eine der vorgenannten Anordnungen umfassend einen rohrförmigen Abschnitt,

wobei die mindestens eine Auslassöffnung (6b) ein vollständig geöffnetes Ende des rohrförmigen Abschnittes ist.

Die vorliegende Offenbarung lehrt ferner eine der vorgenannten Anordnungen umfassend eine erste und eine zweite Seite und einen rohrförmigen Abschnitt, wobei die zweite Seite parallel zur ersten Seite verläuft.

Die vorliegende Offenbarung lehrt ferner eine der vorgenannten Anordnungen,

wobei das Gehäuse (1) an seinem zweiten Ende (1b) einen schrägen Abschnitt umfasst; und
wobei die mindestens eine Auslassöffnung (6c) Teil des schrägen Abschnittes ist.

Die vorliegende Offenbarung lehrt zudem eine der vorgenannten Anordnungen umfassend einen schrägen Abschnitt,

wobei das Gehäuse (1) eine erste Seite umfasst, welche sich vom ersten Ende (1a) des Gehäuses (1) zum zweiten Ende (1b) des Gehäuses (1) erstreckt;
wobei mindestens eine Einlassöffnung der Vielzahl an Einlassöffnungen (4a - 4f) an der ersten Seite angeordnet ist;
wobei das Gehäuse (1) an seinem zweiten Ende (1b) einen schrägen Abschnitt, der schräg zur ersten Seite verläuft, umfasst; und
wobei die mindestens eine Auslassöffnung (6c) Teil des schrägen Abschnittes ist. Gemäss einer Ausführungsform verläuft der schräge Abschnitt in einem spitzen Winkel zur ersten Seite. Gemäss einer anderen Ausführungsform verläuft der schräge Abschnitt in einem stumpfen Winkel zur ersten Seite.

Die vorliegende Offenbarung lehrt weiterhin eine der vorgenannten Anordnungen umfassend einen schrägen Abschnitt,

wobei das Gehäuse (1) eine erste Seite umfasst, welche sich vom ersten Ende (1a) des Gehäuses (1) zum zweiten Ende (1b) des Gehäuses (1) erstreckt;
wobei das Gehäuse (1) eine zweite Seite umfasst, welche sich vom ersten Ende (1a) des Gehäuses (1) zum zweiten Ende (1b) des Gehäuses (1) erstreckt;
wobei die zweite Seite verschieden ist von der ersten Seite;
wobei mindestens eine Einlassöffnung der Vielzahl an Einlassöffnungen (4a - 4f) an der ersten Seite angeordnet ist;
wobei das Gehäuse (1) an seinem zweiten Ende (1b) einen schrägen Abschnitt, der schräg zur ersten Seite und zur zweiten Seite verläuft, umfasst; und
wobei die mindestens eine Auslassöffnung (6c) Teil des schrägen Abschnittes ist.

Gemäss einer Ausführungsform verläuft der schräge Abschnitt in einem spitzen Winkel zur ersten Seite und zur zweiten Seite. Gemäss einer anderen Ausführungsform verläuft der schräge Abschnitt in einem stumpfen Winkel zur ersten Seite und zur zweiten Seite.

Die vorliegende Offenbarung lehrt ferner eine der vorgenannten Anordnungen umfassend eine erste und eine zweite Seite und einen schrägen Abschnitt, wobei die zweite Seite parallel zur ersten Seite verläuft.

Die vorliegende Offenbarung lehrt ebenfalls eine Verbrennungsvorrichtung umfassend einen Feuerraum und einen Abgaskamin und/oder Rauchgaskamin und/oder Schornstein,

> wobei der Abgaskamin und/oder Rauchgaskamin und/oder Schornstein mit dem Feuerraum in Fluidverbindung ist;
> wobei der Abgaskamin und/oder Rauchgaskamin und/oder Schornstein eine Wand (2) umfasst; und
> wobei die Befestigungsvorrichtung (3) einer Anordnung gemäss einem der Ansprüche 1 bis 14 an der Wand (2) mechanisch befestigt ist.

Vorzugsweise umfasst die Wand (2) eine Innenwand und/oder eine Seitenwand. Idealerweise ist die Wand (2) eine Innenwand und/oder eine Seitenwand.

In einer Ausführungsform ist der Feuerraum der Verbrennungsvorrichtung über den Abgaskamin und/oder Rauchgaskamin und/oder Schornstein und über mindestens eine Einlassöffnung der Vielzahl an Einlassöffnungen (4a - 4f) in Fluidverbindung mit der Sensoreinheit (8).

Das Genannte bezieht sich auf einzelne Ausführungsformen der Offenbarung. Verschiedene Änderungen an den Ausführungsformen können vorgenommen werden, ohne von der zu Grunde liegenden Idee abzuweichen und ohne den Rahmen dieser Offenbarung zu verlassen. Der Gegenstand der vorliegenden Offenbarung ist definiert über die nachfolgenden

Ansprüche. Es können verschiedenste Änderungen an den Ausführungsformen vorgenommen werden, ohne den Schutzbereich der folgenden Ansprüche zu verlassen.

**Patentansprüche**

1. Anordnung umfassend eine Sensoreinheit (8) und ein Gehäuse (1) mit einem ersten (1a) und einem zweiten Ende (1b), mit einer Vielzahl an Einlassöffnungen (4a - 4f) und mit mindestens einer Auslassöffnung (6a - 6c);

> wobei die Anordnung am ersten Ende (1a) des Gehäuses (1) eine Befestigungsvorrichtung (3) zur mechanischen Befestigung des Gehäuses (1) an einer Wand (2) umfasst, sodass das Gehäuse (1) gegenüber der Wand (2) im Wesentlichen unbeweglich ist;
> wobei das erste Ende (1a) verschieden ist vom zweiten Ende (1b) und das erste Ende (1a) dem zweiten Ende (1b) gegenüberliegt und die mindestens eine Auslassöffnung (6a - 6c) verschieden ist von jeder Einlassöffnung der Vielzahl an Einlassöffnungen (4a - 4f);
> wobei die Sensoreinheit (8) innen im Gehäuse (1) angeordnet ist und einen ersten Abstand vom ersten Ende (1a) und einen zweiten Abstand vom zweiten Ende aufweist;
> wobei der erste Abstand der Sensoreinheit (8) vom ersten Ende (1a) grösser ist als der zweite Abstand der Sensoreinheit (8) vom zweiten Ende (1b);
> wobei die mindestens eine Auslassöffnung (6a - 6c) einen ersten Abstand vom ersten Ende (1a) und einen zweiten Abstand vom zweiten Ende (1b) aufweist; und
> wobei der erste Abstand der mindestens einen Auslassöffnung (6a - 6c) vom ersten Ende (1a) grösser ist als der zweite Abstand der mindestens einen Auslassöffnung (6a - 6c) vom zweiten Ende (1b).

2. Die Anordnung gemäss Anspruch 1,

> wobei die Befestigungsvorrichtung (3) einen ersten Abstand vom ersten Ende (1a) und einen zweiten Abstand vom zweiten Ende aufweist; und
> wobei der erste Abstand der Befestigungsvorrichtung (3) vom ersten Ende (1a) kleiner ist als der zweite Abstand der Befestigungsvorrichtung (3) vom zweiten Ende (1b).

3. Die Anordnung gemäss einem der Ansprüche 1 bis 2,
   wobei die Befestigungsvorrichtung (3) einen Flansch umfasst.

4. Die Anordnung gemäss einem der Ansprüche 1 bis 3,

> wobei die Anordnung ein Paar elektrischer Leitungen (9a, 9b) umfasst; und
> wobei sich mindestens eine elektrische Leitung des Paares elektrischer Leitungen (9a, 9b) von der Sensoreinheit (8) zur Befestigungsvorrichtung (3) erstreckt.

5. Die Anordnung gemäss Anspruch 4,
   wobei mindestens eine elektrische Leitung des Paares elektrischer Leitungen (9a, 9b) die Sensoreinheit (8) gegenüber dem Gehäuse (1) mechanisch stützt.

6. Die Anordnung gemäss einem der Ansprüche 4 bis 5,

> wobei die Anordnung eine Auswerteeinheit umfasst; und

wobei mindestens eine elektrische Leitung des Paares elektrischer Leitungen (9a, 9b) die Sensoreinheit (8) mit der Auswerteeinheit elektrisch verbindet.

7. Die Anordnung gemäss einem der Ansprüche 1 bis 6,
wobei die Anordnung genau eine Auslassöffnung (6a - 6c) umfasst.

8. Die Anordnung gemäss einem der Ansprüche 1 bis 7,

wobei das Gehäuse (1) eine erste Seite umfasst, welche sich vom ersten Ende (1a) des Gehäuses (1) zum zweiten Ende (1b) des Gehäuses (1) erstreckt;
wobei das Gehäuse (1) eine zweite Seite umfasst, welche sich vom ersten Ende (1a) des Gehäuses (1) zum zweiten Ende (1b) des Gehäuses (1) erstreckt;
wobei die zweite Seite verschieden ist von der ersten Seite;
wobei mindestens eine Einlassöffnung der Vielzahl an Einlassöffnungen (4a - 4f) an der ersten Seite angeordnet ist; und
wobei die mindestens eine Auslassöffnung (6a) an der zweiten Seite angeordnet ist.

9. Die Anordnung gemäss Anspruch 8,
wobei jede Einlassöffnung der Vielzahl an Einlassöffnungen (4a - 4f) an der ersten Seite angeordnet ist.

10. Die Anordnung gemäss einem der Ansprüche 1 bis 7,
wobei die mindestens eine Auslassöffnung (6b) unmittelbar am zweiten Ende (1b) angeordnet ist.

11. Die Anordnung gemäss Anspruch 10,

wobei das Gehäuse (1) eine erste Seite umfasst, welche sich vom ersten Ende (1a) des Gehäuses (1) zum zweiten Ende (1b) des Gehäuses (1) erstreckt;
wobei das Gehäuse (1) eine zweite Seite umfasst, welche sich vom ersten Ende (1a) des Gehäuses (1) zum zweiten Ende (1b) des Gehäuses (1) erstreckt;
wobei die zweite Seite verschieden ist von der ersten Seite;
wobei mindestens eine Einlassöffnung der Vielzahl an Einlassöffnungen (4a - 4f) an der ersten Seite angeordnet ist; und
wobei die mindestens eine Auslassöffnung (6b) ausserhalb der ersten Seite und ausserhalb der zweiten Seite angeordnet ist.

12. Die Anordnung gemäss einem der Ansprüche 10 bis 11,

wobei das Gehäuse (1) an seinem zweiten Ende (1b) einen rohrförmigen Abschnitt umfasst; und
wobei die mindestens eine Auslassöffnung (6b) ein offenes Ende des rohrförmigen Abschnittes umfasst.

13. Die Anordnung gemäss einem der Ansprüche 1 bis 7,

wobei das Gehäuse (1) an seinem zweiten Ende (1b) einen schrägen Abschnitt umfasst; und
wobei die mindestens eine Auslassöffnung (6c) Teil des schrägen Abschnittes ist.

14. Die Anordnung gemäss Anspruch 13,

wobei das Gehäuse (1) eine erste Seite umfasst, welche sich vom ersten Ende (1a) des Gehäuses (1) zum zweiten Ende (1b) des Gehäuses (1) erstreckt;
wobei mindestens eine Einlassöffnung der Vielzahl an Einlassöffnungen (4a - 4f) an der ersten Seite angeordnet ist;
wobei das Gehäuse (1) an seinem zweiten Ende (1b) einen schrägen Abschnitt, der schräg zur ersten Seite verläuft, umfasst; und
wobei die mindestens eine Auslassöffnung (6c) Teil des schrägen Abschnittes ist.

**Claims**

1. Arrangement comprising a sensor unit (8) and a housing (1) with a first (1a) and a second end (1b), with a plurality of inlet openings (4a - 4f) and with at least one outlet opening (6a - 6c);

wherein the arrangement comprises an attachment apparatus (3) for mechanical attachment of the housing (1) to a wall (2) at the first end (1a) of the housing (1), so that the housing (1) is essentially immovable in relation to the wall (2);
wherein the first end (1a) is different from the second end (1b) and the first end (1a) lies opposite the second end (1b) and the at least one outlet opening (6a - 6c) is different from each inlet opening of the plurality of inlet openings (4a - 4f);
wherein the sensor unit (8) is arranged in the inside of the housing (1) and is at a first distance from the first end (1a) and at a second distance from the second end;
wherein the first distance between the sensor unit (8) and the first end (1a) is greater than the second distance between the sensor unit (8) and the second end (1b);

wherein the at least one outlet opening (6a - 6c) is at a first distance from the first end (1a) and at a second distance from the second end (1b); and

wherein the first distance between the at least one outlet opening (6a - 6c) and the first end (1a) is greater than the second distance between the at least one outlet opening (6a - 6c) and the second end (1b).

2. The arrangement according to claim 1,

   wherein the attachment apparatus (3) is at a first distance from the first end (1a) and at a second distance from the second end; and

   wherein the first distance between the attachment apparatus (3) and the first end (1a) is less than the second distance between the attachment apparatus (3) and the second end (1b).

3. The arrangement according to any of the claims 1 to 2,
   wherein the attachment apparatus (3) comprises a flange.

4. The arrangement according to any of the claims 1 to 3,

   wherein the arrangement comprises a pair of electrical leads (9a, 9b); and

   wherein at least one electrical lead of the pair of electrical leads (9a, 9b) extends from the sensor unit (8) to the attachment apparatus (3).

5. The arrangement according to claim 4,
   wherein at least one electrical lead of the pair of electrical leads (9a, 9b) mechanically supports the sensor unit (8) in relation to the housing (1).

6. The arrangement according to any of the claims 4 to 5,

   wherein the arrangement comprises an evaluation unit; and

   wherein at least one electrical lead of the pair of electrical leads (9a, 9b) connects the sensor unit (8) electrically to the evaluation unit.

7. The arrangement according to any of the claims 1 to 6,
   wherein the arrangement comprises precisely one outlet opening (6a - 6c).

8. The arrangement according to any of the claims 1 to 7,

   wherein the housing (1) comprises a first side, which extends from the first end (1a) of the housing (1) to the second end (1b) of the housing (1);

   wherein the housing (1) comprises a second side, which extends from the first end (1a) of the housing (1) to the second end (1b) of the housing (1);

   wherein the second side is different from the first side; wherein at least one inlet opening of the plurality of inlet openings (4a - 4f) is arranged on the first side; and

   wherein the at least one outlet opening (6a) is arranged on the second side.

9. The arrangement according to claim 8,
   wherein each inlet opening of the plurality of inlet openings (4a - 4f) is arranged on the first side.

10. The arrangement according to any of the claims 1 to 7,
    wherein the at least one outlet opening (6b) is arranged directly at the second end (1b).

11. The arrangement according to claim 10,

    wherein the housing (1) comprises a first side, which extends from the first end (1a) of the housing (1) to the second end (1b) of the housing (1);

    wherein the housing (1) comprises a second side, which extends from the first end (1a) of the housing (1) to the second end (1b) of the housing (1);

    wherein the second side is different from the first side;

    wherein at least one inlet opening of the plurality of inlet openings (4a - 4f) is arranged on the first side; and

    wherein the at least one outlet opening (6b) is arranged outside the first side and outside the second side.

12. The arrangement according to any of the claims 10 to 11,

    wherein the housing (1) comprises a tubular section at its second end (1b); and

    wherein the at least one outlet opening (6b) comprises an open end of the tubular section.

13. The arrangement according to any of the claims 1 to 7,

    wherein the housing (1) comprises a slanted section at its second end (1b); and

    wherein the at least one outlet opening (6c) is part of the slanted section.

**14.** The arrangement according to claim 13,

wherein the housing (1) comprises a first side, which extends from the first end (1a) of the housing (1) to the second end (1b) of the housing (1);

wherein at least one inlet opening of the plurality of inlet openings (4a - 4f) is arranged on the first side;

wherein, at its second end (1b), the housing (1) comprises a slanted section, which runs at an angle to the first side; and

wherein the at least one outlet opening (6c) is part of the slanted section.

## Revendications

**1.** Disposition comprenant une unité de détection (8) et un boîtier (1) avec une première (1a) et une seconde extrémité (1b), avec une pluralité d'ouvertures d'admission (4a-4f) et avec au moins une ouverture d'évacuation (6a-6c) ;

dans laquelle la disposition comprend au niveau de la première extrémité (1a) du boîtier (1) un dispositif de fixation (3) destiné à la fixation mécanique du boîtier (1) au niveau d'une paroi (2), de sorte que le boîtier (1) est sensiblement immobile par rapport à la paroi (2) ;

dans laquelle la première extrémité (1a) est différente de la seconde extrémité (1b) et la première extrémité (1a) fait face à la seconde extrémité (1b) et l'au moins une ouverture d'évacuation (6a-6c) est différente de chaque ouverture d'admission de la pluralité d'ouvertures d'admission (4a-4f) ;

dans laquelle l'unité de détection (8) est disposée à l'intérieur dans le boîtier (1) et présente un premier écart par rapport à la première extrémité (1a) et un second écart par rapport à la seconde extrémité ;

dans laquelle le premier écart de l'unité de détection (8) par rapport à la première extrémité (1a) est supérieur au second écart de l'unité de détection (8) par rapport à la seconde extrémité (1b) ;

dans laquelle l'au moins une ouverture d'évacuation (6a-6c) présente un premier écart par rapport à la première extrémité (1a) et un second écart par rapport à la seconde extrémité (1b) ; et

dans laquelle le premier écart de l'au moins une ouverture d'évacuation (6a-6c) par rapport à la première extrémité (1a) est supérieur au second écart de l'au moins une ouverture d'évacuation (6a-6c) par rapport à la seconde extrémité (1b).

**2.** La disposition selon la revendication 1,

dans laquelle le dispositif de fixation (3) présente un premier écart par rapport à la première extrémité (1a) et un second écart par rapport à la seconde extrémité ; et

dans laquelle le premier écart du dispositif de fixation (3) par rapport à la première extrémité (1a) est inférieur au second écart du dispositif de fixation (3) par rapport à la seconde extrémité (1b).

**3.** La disposition selon l'une quelconque des revendications 1 à 2,
dans laquelle le dispositif de fixation (3) comprend une bride.

**4.** La disposition selon l'une quelconque des revendications 1 à 3,

dans laquelle la disposition comprend une paire de fils électriques (9a, 9b) ; et

dans laquelle au moins un fil électrique de la paire de fils électriques (9a, 9b) s'étend de l'unité de détection (8) au dispositif de fixation (3).

**5.** La disposition selon la revendication 4,
dans laquelle au moins un fil électrique de la paire de fils électriques (9a, 9b) soutient mécaniquement l'unité de détection (8) par rapport au boîtier (1).

**6.** La disposition selon l'une quelconque des revendications 4 à 5,

dans laquelle la disposition comprend une unité d'évaluation ; et

dans laquelle au moins un fil électrique de la paire de fils électriques (9a, 9b) relie électriquement l'unité de détection (8) à l'unité d'évaluation.

**7.** La disposition selon l'une quelconque des revendications 1 à 6,
dans laquelle la disposition comprend exactement une ouverture d'évacuation (6a-6c).

**8.** La disposition selon l'une quelconque des revendications 1 à 7,

dans laquelle le boîtier (1) comprend une première face qui s'étend de la première extrémité (1a) du boîtier (1) à la seconde extrémité (1b) du boîtier (1) ;

dans laquelle le boîtier (1) comprend une seconde face qui s'étend de la première extrémité (1a) du boîtier (1) à la seconde extrémité (1b) du boîtier (1) ;

dans laquelle la seconde face est différente de la première face ;

dans laquelle au moins une ouverture d'admission de la pluralité d'ouvertures d'admission (4a-4f) est disposée au niveau de la première face ; et

dans laquelle l'au moins une ouverture d'évacuation (6a) est disposée au niveau de la seconde face.

9. La disposition selon la revendication 8, dans laquelle chaque ouverture d'admission de la pluralité d'ouvertures d'admission (4a-4f) est disposée au niveau de la première face.

10. La disposition selon l'une quelconque des revendications 1 à 7, dans laquelle l'au moins une ouverture d'évacuation (6b) est disposée directement au niveau de la seconde extrémité (1b).

11. La disposition selon la revendication 10,

dans laquelle le boîtier (1) comprend une première face qui s'étend de la première extrémité (1a) du boîtier (1) à la seconde extrémité (1b) du boîtier (1) ;

dans laquelle le boîtier (1) comprend une seconde face qui s'étend de la première extrémité (1a) du boîtier (1) à la seconde extrémité (1b) du boîtier (1) ;

dans laquelle la seconde face est différente de la première face ;

dans laquelle au moins une ouverture d'admission de la pluralité d'ouvertures d'admission (4a-4f) est disposée au niveau de la première face ; et

dans laquelle l'au moins une ouverture d'évacuation (6b) est disposée à l'extérieur de la première face et à l'extérieur de la seconde face.

12. La disposition selon l'une quelconque des revendications 10 à 11,

dans laquelle le boîtier (1) comprend une section tubulaire au niveau de sa seconde extrémité (1b) ; et

dans laquelle l'au moins une ouverture d'évacuation (6b) comprend une extrémité ouverte de la section tubulaire.

13. La disposition selon l'une quelconque des revendications 1 à 7,

dans laquelle le boîtier (1) comprend une section inclinée au niveau de sa seconde extrémité (1b) ;

dans laquelle l'au moins une ouverture d'évacuation (6c) fait partie de la section inclinée.

14. La disposition selon la revendication 13,

dans laquelle le boîtier (1) comprend une première face qui s'étend de la première extrémité (1a) du boîtier (1) à la seconde extrémité (1b) du boîtier (1) ;

dans laquelle au moins une ouverture d'admission de la pluralité d'ouvertures d'admission (4a-4f) est disposée au niveau de la première face ;

dans laquelle le boîtier (1) comprend au niveau de sa seconde extrémité (1b) une section inclinée qui s'étend de manière inclinée par rapport à la première face ; et

dans laquelle l'au moins une ouverture d'évacuation (6c) fait partie de la section inclinée.

FIG 1

FIG 2

FIG 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 2685875 Y
- WO 2022064271 A1
- DE 102012211039 A1
- US 6015533 A
- US 2010050738 A1